# EUROPEAN PATENT APPLICATION

(11) **EP 0 812 789 A2**
(43) Date of publication of application: **17.12.1997**
(21) Application number: 97303982.9
(22) Date of filing: 09.06.1997
(51) Int. Cl.: B65G 47/84

(54) **Device for altering the spacing between conveyed articles**

(30) Priority: 14.06.1996 US 663643
(71) Applicant: Curt G. Joa, Inc., Sheboygan Falls, Wisconsin 53085-0903 (US)
(72) Inventor: Meyer, Thomas C., Elkhart Lake, Wisconsin 53020 (US)
(74) Representative: MacGregor, Gordon

(57) **Abstract**

Articles being conveyed on a source conveyor belt with uniform spacing between them are deposited on a receiving conveyor belt with a different uniform spacing between them utilizing a device that has one or more transfer units mounted for being driven rotationally about a common axis, each transfer unit having axially extending article pickup members which all rotate or orbit in the same circular path for gripping successive articles being presented on the source conveyor and transferring the articles to the receiving conveyor. The transfer units are driven rotationally with individual servomotors which are controlled to accelerate and decelerate cyclically such that the article pickup members arrive at the source conveyor or moving at the same linear speed as the source conveyor to pickup an article and arrive at the receiving conveyor moving linearly at the same speed as the receiving conveyor to deposit the article without relative motion between the pickup members and the conveyors while picking up and while depositing the articles.

## Description

### Background of the Invention

The invention disclosed herein pertains to a device for gripping articles being transported on a conveyor with a first pitch or spacing between them and transferring the articles to a second conveyor that is moving at a speed that differs from the speed of the first conveyor so the articles will have a different pitch or spacing between them on the second conveyor.

In manufacturing certain products such as disposable diapers, sanitary napkins and similar products, it becomes necessary occasionally to make parts for products at one location, to deposit the parts consecutively on a conveyor with the parts moving at one pitch spacing, and to transfer the parts to a second conveyor moving at a second pitch spacing for the parts to be conveyed to a product assembly operation that requires having the parts received at the second pitch spacing. Accurate positioning of the part is especially important when the part being handled is a delicate light weight part such as the fluffy absorption pad that is fabricated into disposable diapers and sanitary napkins although positional accuracy and reliable gripping of the product is important in other manufacturing operations as well. A frequently utilized but not wholly satisfactory method of effecting a pitch or article spacing change is to arrange two conveyors in tandem, one above the other, and let the articles on the upper conveyor that is running at one speed drop or spill the articles onto the other conveyor that is running at another speed. There is obvious loss of control over the article as it executes a free fall from the upper conveyor to the lower conveyor. Another line of preexisting pitch changing devices is notable for complexity and inflexibility by virtue of having a plurality of circumferentially spaced apart arms extending radially outwardly from a drum and adapted to pick up an article from one conveyor and transfer it to another where there is a cam in the drum for driving the arms in and out to change the radius of the arms as the drum rotates. Preexisting article pitch changing devices and conveyor to conveyor article transferring devices are disclosed in U.S. Patents 4,726,876 and 4,880,102.

### Summary of the Invention

The new transfer device disclosed herein is notable for its precise and enduring control of an article from the moment it is picked up from a conveyor moving articles at one pitch spacing to the moment the article is released to a conveyor for moving articles at a different pitch spacing. The device is also distinguished by its flexibility and adaptability for cooperating with article source and article receiving conveyors that run at a variety of selectable speeds and with a wide selection of pitch spacings chosen for the source and receiving conveyors. Articles are transferred from one conveyor to another with at least one transfer unit or more than one transfer unit, each rotating about a single axis. Each article transfer unit is driven rotationally independently of another unit with individual servomotors that are controlled by a programmable controller. This affords an opportunity to control the angular velocity and position profiles of the rotating article transfer units during every rotation so the units remain in precise phase relationship with the article source and article receiving conveyors even though the conveyors run at substantially different velocities in a particular installation and in a variety of conveyor installations. An illustrative embodiment of the invention comprises two u-shaped rotary transfer units each of which has two pickup heads that are formed diametrically opposite of each other on opposite sides of an axis of rotation about which both of the transfer units rotate in the same circular path. The pickup heads of both transfer units rotate in the same orbital path and in controlled phase relationship relative to each other such that the heads on one transfer unit are prohibited from colliding with the heads on the other unit. The transfer units are driven rotationally, coaxially and independently of each other with separate motors which are jointly controlled to thereby maintain the prescribed but varying phase relationship between the orbiting heads on the two units at all times. The transfer units are, for example, preferably driven rotationally and independently with individual servomotors under the control of a programmable microprocessor based controller although the relative velocity of the pickup heads of the rotary transfer units could also be controlled utilizing any of several known mechanical devices including indexers, which vary the velocity of an output shaft relative to the velocity of an input shaft in accordance with a pre-planned profile. As indicated above, however, utilizing servomotors which are programmed to simulate a cam profile is preferred because of the ease with which the device can be reprogrammed to yield a profile that is particularized for dealing with various conveyed article pitches.

Control of the two rotating units is such that when one of the transfer units is altering its rotational velocity for a pickup head thereon to match the velocity and timing of the source conveyor from which an article is to be picked up, the other of the transfer units is altering its velocity for a head thereon to match the velocity of the receiving conveyor on which the article is to be deposited so there is no motion of a pickup head relative to the article while being picked up or deposited. Because there are two diametrically opposite pickup heads on each transfer unit, each unit picks up two articles per revolution. Thus, one unit picks up the first and third articles in the succession of articles on the source conveyor and transfers the articles to the receiving conveyor and the other unit picks up and transfers the second and fourth articles from the source conveyor. An article picked up in one quadrant of rotation is deposited in the next quadrant of rotation.

As a pickup head on a transfer unit approaches the position wherein it is about to pick up an article in the illustrative embodiment described herein, a vacuum commutator connects the head to a source of vacuum to provide the means for gripping the article. When the pickup head carrying the article approaches the receiving conveyor, the article is pressed to this conveyor and the vacuum is shut off, allowing the article to be released to the receiving conveyor. With some articles, a pulse of pressurized air is used to break any residual adherence the article may have to the head after the vacuum is cut off. Various means other then vacuum can be used to enable a head to engage with an article and to release it.

How the foregoing objectives and features of the new article spacing altering device are implemented will be evident in the ensuing more detailed description of a preferred embodiment of the invention which will now be set forth in reference to the accompanying drawings.

### Description of the Drawings

FIGURE 1 is a diagram that is useful for presenting an overview of the new device for altering the spacing between conveyed articles;
FIGURE 2 is a transverse section visualized by looking in the direction of the arrows 2-2 in the FIGURE 5 top view of the device with the rear of the device being in the leftmost region of the figure;
FIGURE 3 is a sectional view taken through one of the vacuum article pickup heads on a line corresponding to 3-3 in FIGURE 2;
FIGURE 4 is a sectional view taken through one of the vacuum pickup heads on a line corresponding to 4-4 in FIGURE 2;
FIGURE 5 is a right side elevational view of the transfer device considering that the left region of the figure is the rear end of the device and with the presently uppermost of the vacuum heads positioned for picking up an article; shown in phantom lines;
FIGURE 6 is a right side elevational view of the new transfer device looking in the direction of the arrows 6-6 in FIGURE 5 considering that the right region of the figure is the front end of the device;
FIGURE 7 is a vertical sectional view of a brace member taken on a line corresponding with 7-7 in FIGURE 5, the member being involved in supporting the transfer device from a machine frame in cantilever fashion and in adjusting the position of the transfer device relative to a conveyor;
FIGURE 8 is a rear end elevational view of the new article spacing device mounted to a machine, for example, a diaper making machine; and

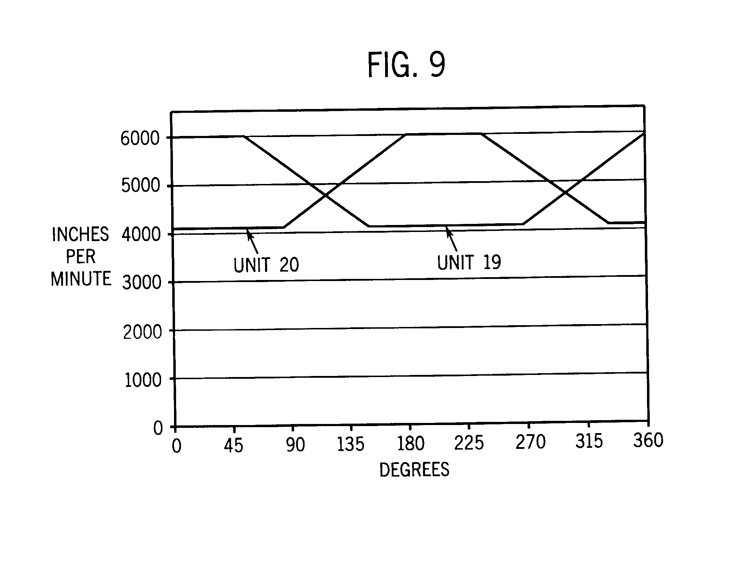

FIGURE 9 is a timing diagram that is useful for explaining the phasing and timing of the concurrently rotating vacuum article pickup and transfer heads.

### Description of a Preferred Embodiment

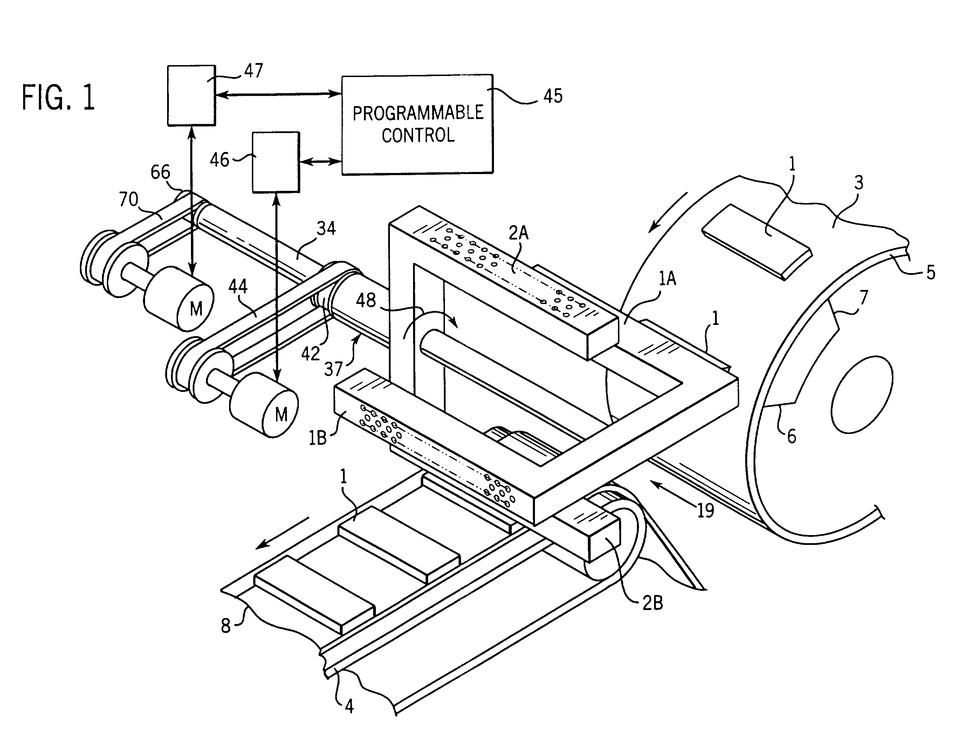

FIGURE 1 affords an opportunity to present an overview of an application of the new article interspace altering device. In this diagram articles 1 are being conveyed on a linear belt conveyor 3 with a uniform space between them. The articles may, for example, be soft and unstable fluff pads that are made by filling suitably shaped circumferentially spaced apart recesses in a rotationally driven drum, not shown, so the articles 1 are discharged from the drum and are deposited on a first conveyor 3 at uniform spacing. The first conveyor 3 can be called the article source conveyor. The pads may be destined to become absorbers in disposable diapers or sanitary napkins, for example. Fabrication of a continuous web of sanitary napkins or disposable diaper articles will usually require that the pads be at a spacing that differs from the spacing on source conveyor 3. So the pads are transferred from the first or source conveyor 3 to the upper run 4 of an article receiving conveyor belt where the articles will have a different spacing. In this example, the article spacing on the second or receiving conveyor 4 is shorter than the article spacing on the first or source conveyor 3. The source conveyor belt 3 runs on a large drum 5 which has a vacuum manifold extending between limits 6 and 7 so articles may be carried around a quadrant of drum 5 to be released, by discontinuance of the vacuum on the drum, to a vacuum head 1A of the one, 19, of the two transfer heads 19 and 20 utilized in the illustrative embodiment of the new space altering device. A web, which may be a thin plastic sheet 8 for a disposable diaper is fed onto conveyor 4. The pad 1, or whatever the article is, can be pressed onto receiving conveyor belt 4 directly or, as in this example, onto a continuous thin plastic backing sheet 8 for a web of diapers that are to be fabricated downstream. The sheet 8 usually has adhesive stripes, not shown, on it for holding the pads at uniform spacing. The backing sheet 8 and the receiving conveyor 4 are translating at the same speed.

The principal components of the new device for altering the spacing between articles 1 that are transferred from a conveyor 3 running at one speed to a conveyor 4 running at another speed are shown diagrammatically in FIGURE 1. Articles are transferred from source conveyor 3 to receiving conveyor 4 with two rotationally driven transfer units which are generally designated by the reference numerals 19 and 20, respectively. Article transfer units 19 and 20 are basically the same insofar as structure and function is concerned. Typical unit 20 has a generally u-shaped configuration and comprises two diametrically opposite article gripping means in the form of axially extending vacuum pickup heads 2A and 2B. Unit 19 has two vacuum pickup heads 1A and 1B. The transfer units 19 and 20 rotate about a common axis which is the axis of the shaft 34. The vacuum heads 1A and 1B of transfer unit 19 are interposed between vacuum heads 2A and 2B of unit 20. Since the units are the same and are coaxial it follows that the vacuum heads 1A and 1B on unit 19 orbit in the same circular path as the vacuum heads 2A and 2B of the other unit 20. The units are driven by individual servomotors marked with the letter M under the control of a programmable controller which is so labeled and marked with the numeral 45. The drivers for interfacing the controller with the motors are marked 46 and 47. The controller keeps the transfer units in phase with the source conveyor even though the units vary in displacement relative to each other. Assume, for example, that in the diagrammatically illustrated application of the device depicted in FIGURE 1, the units are turning in the direction indicated by the arrow 48 and that the articles 1 are to be transferred from source conveyor belt 3 to receiving conveyor belt 4. First of all recognize that the controller 45 is programmable so it can cope with situations where conveyor belt 3 or 4 is the fast or slow conveyor and with selecting if the space between the articles 1 on the receiving conveyor 4 are to have more space between them or less space between them than they have on the source conveyor 3. In FIGURE 1, for example, the timing is such that transfer unit 20 could be accelerating or decelerating so that when article pickup head 2A lines up with article 1 on source conveyor 3 another head 1A is moving at the exact speed of the source conveyor belt 3. Then head 2B, which is carrying an article 1 continues rotation and accelerates or decelerates to an appropriate speed so it will match the speed of the receiving conveyor 4. Thus, there is vacuum release and the article is deposited on the receiving conveyor when there is no relative motion between the article on the head and the conveyor belt 4. Meanwhile, article handling head 1B on unit 19 would have just left the receiving conveyor belt 4 after just having deposited the article on it. Then unit 19 will accelerate or decelerate so that it arrives at where it can pick up an article from source conveyor 3 and have its speed match the source conveyor belt speed.

In the generalized diagram of the article re-spacing device shown in FIGURE 1, it should be recognized that servomotor driven shaft 34 is connected directly to transfer unit 19 and unit 19 is driven rotationally and is alternately accelerated and decelerated during each revolution of the unit. Transfer unit 20 on the other hand, is journaled for rotation on shaft 34 and the bearings for having transfer unit 20 rotate on shaft are in a sleeve that is generally designated by the numeral 37. The sleeve 37 and bearings are shown in detail in figures which will be discussed later. Sleeve 37 is fastened to transfer unit 20. Sleeve 37 has a pulley 42 fastened to it. The pulley and, hence, unit 20 are driven by a belt 44 under the influence of a servomotor M. The bearings on which shaft 34 is journaled are not shown in the FIGURE 1 diagram but will be shown and described in detail shortly hereinafter.

It will be appreciated by those skilled in the art after considering a transfer device using two transfer units 19 and 20 as described in detail herein that is some applications only one transfer unit could be used. One transfer unit could employ the feature of driving the unit rotationally with a reluctance motor or a servomotor. The concept of controlling angular position and velocity of the transfer unit can be applied so that the peripheral velocity of an article securing head matches the velocity of source conveyor at one angular article pickup position and matches the velocity of the receiver conveyor at an article release position.

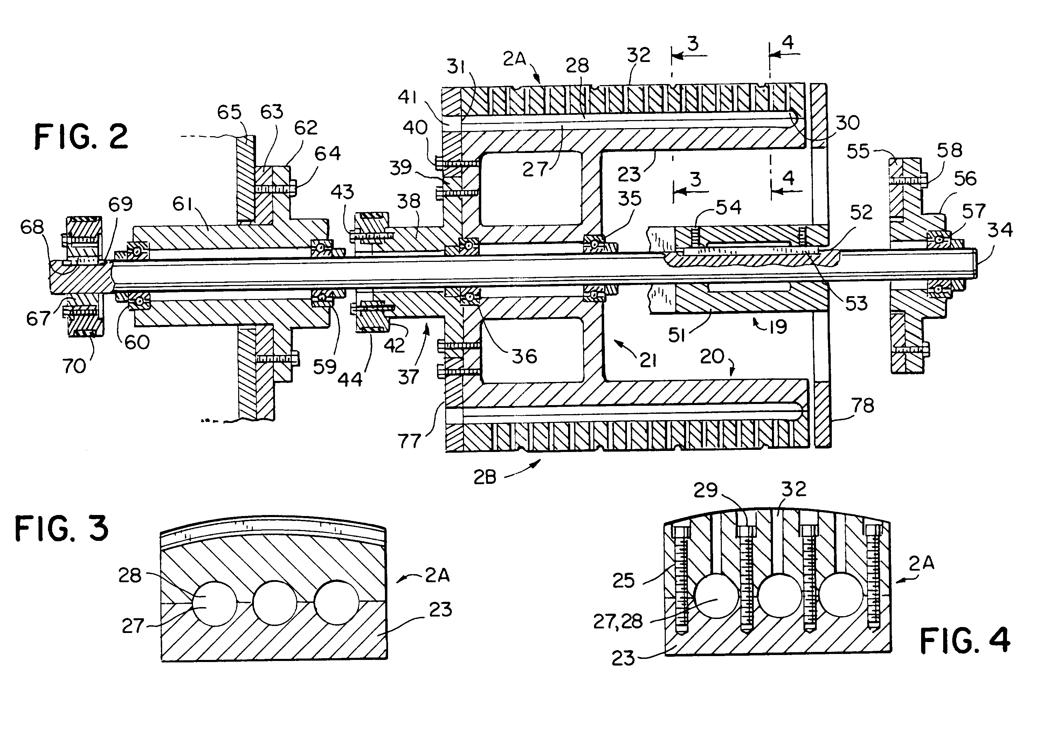

Attention is now invited to the vertical section of the article re-spacing device shown in FIGURE 2. Some parts are omitted from this figure to avoid obscuring parts that are now to be described. The illustrated version of the device utilizes the two independently rotatable article transfer units, one, 20, of which appears in section in FIGURE 2 and the other, 19, is shown fragmentarily and in section. The typical unit 20 is comprised of a frame 21 which has circumferentially grooved diametrically opposite axially extending vacuum article grasping heads 2A and 2B. A vacuum head member, marked 2A to correspond with FIGURE 1, is mounted to base member 23 by means of a plurality of machine screws 29 as shown in FIGURE 4. As is apparent in FIGURES 2, 3 and 4, base member 23 and vacuum head member 2A have semicircular axially extending grooves 27 and 28, respectively. These grooves, when they are superposed, form channels which are circular in cross section as is evident from inspection of FIGURES 3 and 4. The typical channel, defined by grooves 27 and 28 which are semicircular appear in cross section and extend axially. The typical channel, defined by grooves 27 and 28 is closed at its remote end 30 and has a vacuum applying port 31 at the opposite end. Typical vacuum head member 2A has a plurality of suction holes 32 which attract and hold an article as it is being transferred from a source conveyor to a receiving conveyor.

As shown in FIGURE 2, frame 21 of unit 20 which supports vacuum heads 2A and 2B is journaled for rotation on a shaft 34 by means of bearings 35 and 36. The previously mentioned hub assembly 37 has a radially extending flange 39. The flange 39 is secured to vacuum head supporting frame 21 by means of a plurality of screws 40. Flange 39 has a port 41 that communicates with vacuum inlet 31 of the vacuum channel 27,28. The manner in which vacuum is selectively applied to port 41 to attract and carry an article 1 and how the vacuum is discontinued from port 41 to release the article will be elaborated later. The hub 37 has a toothed pulley 42 fastened to its end by means of screws 43. The toothed belt for driving the hub 37, frame 21 and the vacuum heads 2A and 2B rotationally as a unit is marked 44. The vacuum head member 2B is identical to diametrically opposite head member 2A so 2B need not be described. The belt 44 is driven through the agency of a servomotor which is not shown in FIGURE 2 but was mentioned when the FIGURE 1 diagram was being discussed. It will evident from the foregoing discussion that frame 21 and everything attached to it is driven rotationally on shaft 34 by pulley 42 but the pulley does not drive the shaft rotationally.

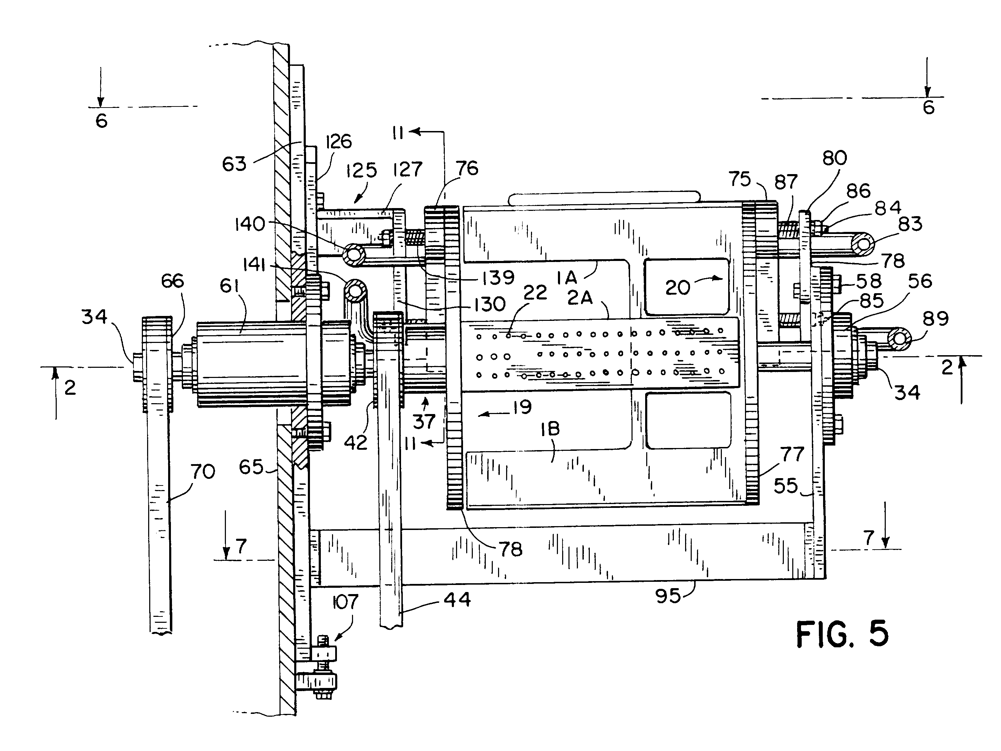

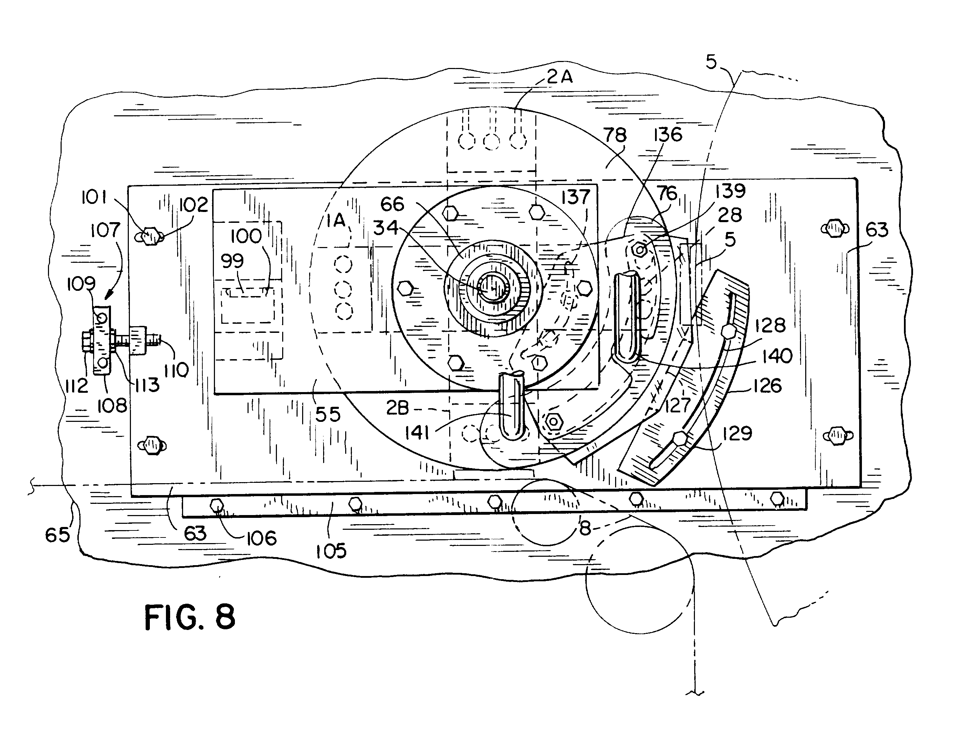

As shown fragmentarily in FIGURE 2 and in the FIGURE 5 top plan view of the device and in other figures, there is another article pickup and transfer unit 19 mounted for rotating about the axis of shaft 34. Unit 19 is substantially the same as unit 20 which was just discussed in reference to FIGURE 2 except that unit 19 is fastened to the shaft 34 so when shaft 34 turns the unit 20 must necessarily turn with it. Referring again to FIGURE 2, one may see that a part of the transfer unit 19 has a collar 51 extending axially from it. This collar is a part of the frame that supports the second vacuum transfer unit 19. The collar has a straight key 52 which registers in a keyway 53 in shaft 34. The key is secured in the keyway by means of screws 54. Shaft 34 is supported at both ends as is apparent in FIGURE 2. At the outboard or front end the shaft is supported in the machine, not shown, in which the device is installed, by a plate 55. A bearing capsule 56 is mounted in the plate 55 and the shaft 34 is in a bearing 57 which is fixed in the capsule. The capsule is fastened to the plate by means of screws 58. At the rear or its inboard end, shaft 34 is journaled by means of bearings 59 and 60 which are mounted in a capsule 61. The capsule has a flange 62 to facilitate mounting it to a rectangular plate 63 which appears in its entirety in FIGURE 8. Plate 63 is fastened by means of screws 64 to a stationary plate 65 which is part of the machine, not shown, in which the article transfer and spacing device is installed. A driving pulley 66 is fixed on the shaft 34 by way of a bushing 67 in which there is key 68 that registers in a keyway 69 in the shaft 34. The pulley 66 has a toothed driving belt 70 meshed with it. The belt is driven by one of the servomotors shown diagrammatically in FIGURE 1.

From the description of the new article re-spacing device thus far set forth, one may see that the diametrically opposite vacuum heads 1A and 1B of unit 19 and the diametrically opposite vacuum heads 2A and 2B of transfer unit 20 are driven with separate motors about the shaft 34 axis for rotating in the same circular path where the motors are controlled to maintain proper angular spacing between the vacuum heads of each rotating unit 19, 20 and the units are further controlled to accelerate and decelerate so the vacuum heads always arrive at an article on a conveyor traveling at the same speed as the article on the conveyor.

It is necessary to turn the vacuum on when a vacuum head 1A, 1B or 2A, 2B is adjacent an article and is ready to remove the article from the source conveyor. It should recognized that one of the conveyors 3 or 4 can act as a source conveyor and the other can act as the receiving conveyor. The transfer units can be driven at speeds that are properly related to the conveyor speeds. It is necessary to turn off the vacuum on the head to release an article to the receiving conveyor. Hence, as shown in FIGURE 5, there are vacuum manifolds 75 and 76 provided for cooperating with the end plates 77 and 78, respectively, on which the vacuum heads 1A,1B and 2A, 2B are mounted. Use of vacuum heads for maintaining suction on a rotating member to hold an article through one angle of rotation and relieving the vacuum and suction to release an article at another angle of rotation is well known in the technology field to which the invention pertains. Transfer unit 20, comprised of vacuum heads 1A and 1B, rotates relative to manifold 75. The stationary manifold 75 is hollow and interfaces in a leakless manner with rotating end plate 77 of transfer unit 20. At the moment, vacuum supplied by way of tube 83 is applied to the channels 28 in head 1A so an article 1 on the source conveyor could be attracted or could be ready to be attracted by the head. The manifold has studs 84 and 85 screwed into it for support. As is typical of both studs, a coil spring 87 is interposed between the manifold 75 and a support plate 78 for the springs to apply sealing force to the manifold against the rotating unit 20. Nuts, such as the one marked 86 are provided to permit adjustment of the spring pressure. The plate 80 is supported from plate 55. Plate 55 is supported indirectly from the frame of the machine in which the device is installed. In some applications of the new article re-spacing device, such as where fluff pads are the articles 1 which the device is handling, it may be necessary to assist removal of the pad even if the vacuum on the pickup head has been cut off from the manifold and the pad should release. Accordingly, a pipe 89 is coupled to a source, not shown, of pressurized air. The pipe is connected to the manifold in such angular relationship that a puff of air is propagated to the channels 28 of the head simultaneously with cut off of the vacuum to thereby eject the pad from the vacuum head.

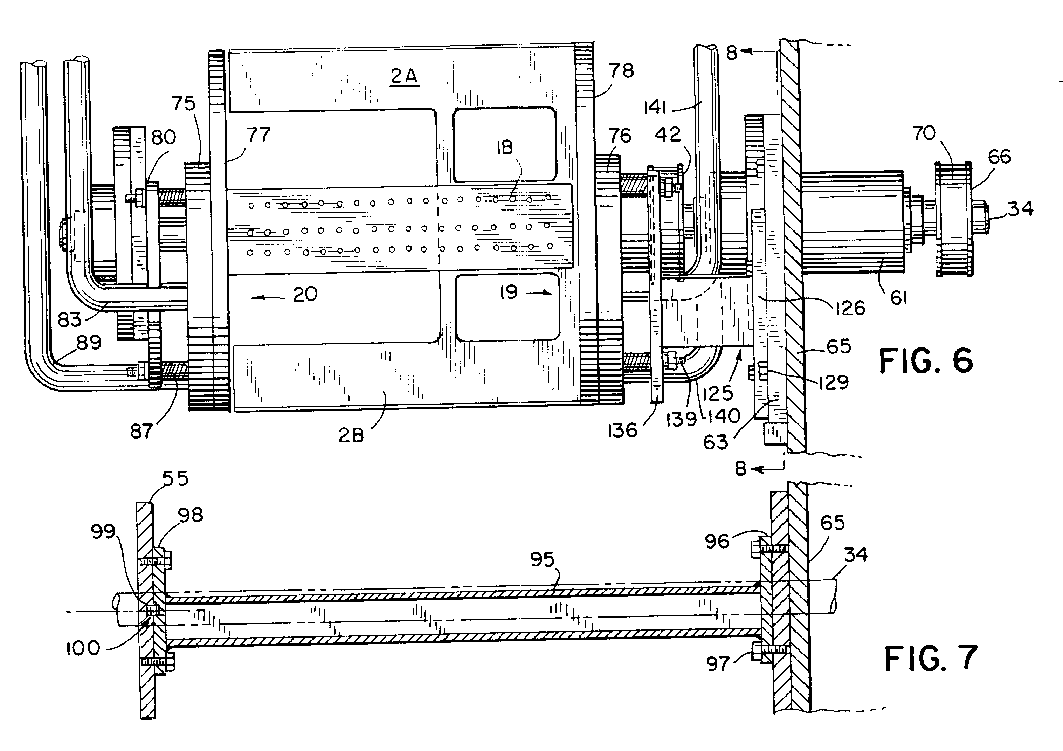

The FIGURE 6 side elevational view of the device is oriented oppositely from FIGURE 5 to illustrate the way in which the vacuum and pressurized air pipes are disposed relative to the device. This figure also depicts how the device is supported at its back end, at the right region of the view of the machine in which the new article re-spacing device is installed. The device is supported from a frame plate 65 of the machine. A tubular member 95 shown in FIGURE 7 serves as a cantilever beam. Member 95 has an end plate 96 welded to it which is secured to plate member 63 by means of machine screws 97. A plate 98 is also welded to the outboard end of beam 95. It is fastened to support plate 55 with machine screws. There is a straight key 99 set in the beam. Key 99 also appears in dashed lines in FIGURE 8 where it is shown to reside in a keyway 100 which is longer than the key so there is free space between the key end and the ends of the keyway for adjustment. The mechanism is supported on plate 55. The beam 95 is fixed to the plate 63 which, in turn, is carried on four machine screws, one of which is marked 101 in FIGURE 8. Screws 101 pass freely through elongated holes 102 in the plate 63. The screws 101 are threaded into machine frame plate 65. Plate 63 is supported on a slide bar 105 which is fixed to stationary machine frame plate 65 by means of machine screws 106. This slightly slidable device support it possible to adjust the position of the rotating article pickup heads relative to the articles on the conveyors to get the best contact with the article that is being grasped from a conveyor by a vacuum pickup head.

An adjustment jack is generally indicated by the numeral 107 in FIGURE 8. The jack comprises a bushing holder 108 which is fixed to the machine plate 65 by means of screws 109. An adjusting screw 110 screws into a threaded block which is fastened to slidable plate 63. The screw 110 has two collars 112 and 113 which restrain it from moving axially when it is turned. To make an adjustment of the position of the article transferring device, the clamping screws 101 are loosened slightly and the adjustment screw 110 is turned in the desired direction to advance or retract the device and then the screws 101 are tightened again.

Views of the rear or inboard end of the article transfer device are presented in FIGURES 5 and 8. These figures show the manifold 76 and the manner in which it provides vacuum to the rotary unit 20 that has the vacuum article gripper members 2A and 2B. In FIGURE 8 the curved manifold 76 is bearing in vacuum tight relationship against the circular plate 78 on which members on which vacuum gripper heads 2A and 2B are fastened. The manifold 76 is supported from a bracket 125 comprised of baseplate 126 and a standoff as seen best in FIGURE 5. Baseplate 126 has an elongated slot to provide the baseplate to be mounted, for being angularly adjusted, to plate 63 with machine screws 129. A plate 136 projects from standoff 127 and facilitates supporting manifold 76. Plate 136 is integral with bracket 126. Plate 136 is slotted at 137 slide adjustably on the machine. Plate 136 has holes through which spring retainer studs 139 extend. Vacuum pipe 140 and blow off pipe 141 connect to manifold 76.

FIGURE 8 is a plot of the velocities of the individual article transfer units 19 and 20 for one revolution of each unit wherein each unit transfers two articles from a source conveyor to a receiving conveyor per revolution. In this particular example, the source conveyor is moving linearly faster than the receiving conveyor. The source conveyor can be converted to the receiving conveyor and the receiving conveyor to the source by reversing the direction of rotation of the transfer units.

In FIGURE 8, within approximately the first 90 degrees of rotation of unit 20, one of its vacuum pickup heads, such as head 2B, has just released an article to the slower conveyor that is moving about 4100 inches per minute by way of example and not limitation. Opposite pickup head 2A is empty but is 180 degrees ahead of 2B so it will be gripping an article from the source conveyor in about 90 degrees more of rotation. When finishing the 90 degrees of rotation, unit 20 begins to accelerate up to about 6,000 inches per minute in this example for pickup head 2A to arrive at the source conveyor to meet an article which is traveling at that speed. An article is then picked up. After leaving the faster source conveyor, head 2A with an article thereon begins to decelerate at about 230 degrees and at about 320 degrees its speed will match the speed of the receiving conveyor. As the diagram indicates, when the unit is decelerating, for a pickup head to arrive at the slower conveyor at a corresponding speed, the other unit is accelerating to arrive at the faster conveyor at a corresponding speed. The total time for each transfer unit to make a full revolution is the sum of its acceleration, deceleration and two constant velocity intervals. Thus, the transfer units do not interfere with each other even though there pickup heads 1A, 1B, 2A, and 2B are orbiting in the same circle.

The actual diameter of the transfer units 19 and 20 is determined by several factors, which include the circumferential displacement required to accommodate the desired pitches. Among these factors are the width of the securing heads 1A, 1B, 2A, and 2B relative to the total circumference. This determines the minimum times spent at the high and low velocities during the transfer periods. This time is subtracted from that part of the total cycle which is available to change velocity, a factor which influences the total force needed to effect the change in velocity. The nominal diameter must allow the heads to maintain the correct relative velocities during the transfer periods and still allow for the correct change in velocity to occur within the required remaining displacement.

To correct for a certain amount of displacement error enforced by the duration of the high and low velocity periods, the remaining "change of velocity" period can be spent as required, with more or less time spent at the high or low velocity to make up for displacement errors. However, this results in less time being available for speed changes and increases the required application of torsional force to overcome inertia.

To accommodate for displacement requirements which exceed the constraints placed on the system by the actual diameter of the transfer units the actual operating speed and the required high and low velocity periods, slight changes in outside diameter can be made to the securing heads. This helps to reduce the slope of the acceleration and deceleration ramps to some manageable level by maximizing the ramp period. The benefit of this lies in the reduction of required torque to more reasonable levels.

Variables in an installation may comprise article source and receiving conveyors running at different speeds or at the same speeds where the rotational speed profile of the transfer units is programmed to achieve a change in article pitch from conveyor to conveyor according to the invention.

## Claims

1. Apparatus for transferring articles from a moving source of said articles to a receiver moving at a velocity differing from the velocity of the moving source, the apparatus comprising:
at least two article transfer units supported for being driven rotationally about a common axis in a position proximate to said source and receiver,
each transfer unit having at least one article engageable and releasable device radially spaced from said axis and projecting axially for said article engageable and releasable device of each transfer unit to be angularly spaced apart and rotating in the same circular path,
individual variable-speed motors operatively coupled to said transfer units, respectively, to drive said transfer units rotationally independently of each other and to accelerate and decelerate said transfer units alternately and successively for said article engageable and releasable devices to arrive at said source to engage an article while moving at the same velocity at which said article is moving and to arrive at said second conveyor and releasing said article while moving at the same velocity that said second conveyor is moving.

2. The apparatus according to claim 1 wherein said article engageable and releasable devices are vacuum heads having a surface containing at least one aperture for engaging an article by vacuum attraction, said heads having an internal passageway with which said aperture communicates and said passageway having an inlet port,
a manifold coupled to a source of vacuum, said port becoming coupled to said manifold at least by the time a head is rotated to a position for engaging an article and said port being decoupled from said manifold at least by the time said head is rotated to a position at which the article is released.

3. Apparatus according to any one of claims 1 or 2 including,
a controller coupled to said motors for controlling said motors to cause said rotating transfer units to accelerate and decelerate separately from each other in such phase relation as to prevent the article engageable and releasable device of one transfer unit from colliding with the said device of another transfer unit.

4. Apparatus according to claim 3 wherein said motors are servomotors and said controller is a programmable controller.

5. Apparatus according to any one of claims 1 or 2 wherein a shaft provides said axis about which said transfer units rotate, and wherein,
one of said transfer units is driven rotationally independently of the other about said shaft by one of said motors and another transfer unit is fastened to said shaft and said shaft is driven rotationally by another of said motors.

6. Apparatus according to claim 3 wherein a shaft provides said axis about which said transfer units rotate and, wherein,
one of said transfer units is driven independently of the other rotates about said shaft by one of said motors and another transfer unit is fastened to said shaft and said shaft is driven rotationally by another of said motors.

7. Apparatus according to claim 3 wherein a shaft provides said axis about which said transfer units rotate,
one of said transfer units is journaled for rotation on said shaft, and a first pulley that is concentric to said shaft is coupled to said one of the transfer units,
another pulley is driven rotationally by a first of said motors and a closed loop flexible member connects the pulley driven by said one motor in driving relation with said first pulley,
the other of said transfer units is fastened to said shaft and a second pulley is concentric to the shaft and coupled to said other of the transfer units,
a pulley is driven by a second one of the motors and a closed loop flexible member connects said last named pulley in driving relation with said second pulley.

8. Apparatus according to any one of claims 1, 2, 3, 4, 5, 6 or 7 wherein said moving source of articles is a belt conveyor and said moving receiver for said articles is a belt conveyor.

9. Apparatus according to claim 8 wherein said articles are uniformly spaced apart in succession on said belt conveyor constituting said moving source.

10. Apparatus for transferring articles from a first conveyor on which the articles are moving in succession with a first uniform spacing between them to a second conveyor on which successive articles are to be moved with a second spacing between them, the apparatus comprising:
at least two article transfer units supported for being driven rotationally about a common axis in a position between said first and second stations,
each transfer unit having at least two article engageable and releasable devices radially spaced from said axis and equiangularly spaced about said axis, said article engageable and releasable devices on one of said transfer units projecting axially in one direction and said devices on another of said transfer units projecting axially in a direction oppositely of said one direction to provide for said article engageable and releasable devices of each transfer unit to be interleaved with each other and rotating in the same circular path,
individual variable-speed motors operatively coupled to said transfer units, respectively, to drive said transfer units rotationally independently of each other and to accelerate and decelerate said transfer units alternately and successively for said article engageable and releasable devices to arrive at said first conveyor to engage an article while moving at the same velocity at which said article is moving and to arrive at said second conveyor and releasing said article while moving at the same velocity at which said second conveyor is moving, and
a controller coupled to said motors for controlling the speed of said variable-speed motors.

11. Apparatus for transferring articles from a moving source of said articles to a receiver moving at a velocity differing from the velocity of the moving source, the apparatus comprising:
a transfer unit supported for being driven rotationally about a common axis in a position proximate to said source and receiver,
said transfer unit having at least one article engageable and releasable device radially spaced from said axis and projecting axially,
a variable-speed motor operatively coupled to said transfer units, respectively, to drive said transfer unit rotationally and to accelerate and decelerate said transfer unit alternately and successively for said article engageable and releasable device to arrive at said source to engage an article while moving at the same velocity at which said article is moving and to arrive at said second conveyor and releasing said article while moving at the same velocity that said second conveyor is moving,
a controller coupled to said variable-speed motor to control the speed of said motor.
